Europäisches Patentamt

European Patent Office

Office européen des brevets

(11) Publication number: **0 040 884**
**B1**

(12) **EUROPEAN PATENT SPECIFICATION**

(45) Date of publication of patent specification: **29.01.86**

(51) Int. Cl.⁴: **A 61 F 2/00, A 61 B 17/56**

(21) Application number: **81200547.8**

(22) Date of filing: **20.05.81**

(54) **Orthopedic coupling device.**

(30) Priority: **23.05.80 NL 8002998**

(43) Date of publication of application:
**02.12.81 Bulletin 81/48**

(45) Publication of the grant of the patent:
**29.01.86 Bulletin 86/05**

(84) Designated Contracting States:
**AT BE CH DE FR GB IT LI LU NL SE**

(56) References cited:
**CH-A- 597 838**
**DE-B-2 621 667**
**FR-A-1 136 123**
**GB-A- 28 864**
**GB-A-2 017 502**
**NL-A-7 805 262**
**US-A-2 801 631**

(73) Proprietor: **Smit, Jan Willem**
**No. 31, Schumannlaan**
**NL-7522 KD Enschede (NL)**

(72) Inventor: **Smit, Jan Willem**
**No. 31, Schumannlaan**
**NL-7522 KD Enschede (NL)**

(74) Representative: **Schumann, Bernard Herman Johan et al**
**OCTROOIBUREAU ARNOLD & SIEDSMA**
**Sweelinckplein 1**
**NL-2517 GK The Hague (NL)**

Courier Press, Leamington Spa, England.

## Description

The invention relates to an orthopedic coupling device comprising a generally U-shaped clamp of resilient material, having two legs extending in spaced and parallel relation.

Such a coupling device is known from e.g. GB—A—2 017 502.

The invention has for its object to provide a coupling device of the above-mentioned type, specifically adapted for the coupling of a technical element, e.g. an endoprothesis, with a bone.

Such a coupling device is well-known in various embodiments. Often use is made of an endoprothesis, for example an artificial elbow, that is provided with two pins extending from the external surfaces of the prothesis and being intended to be used with or without cement or bone grafts, to be fitted into longitudinal holes, which are present or made in the bones concerned. In this way a firm short-term fixation is obtained; namely in practice it often appears that by the unnatural loading of the bone and among others by temperature effects of the curing of the bone cement, so called "resorption" occurs, causing the bone to move backward and loosening the initial firm coupling.

Screw and bolts are often used as coupling devices. A screw for example is put through the bone wall, into the prothesis; a bolt can be put through the complete bone in a transverse direction, the head of the bolt extending at one side of the bone and the free end with the fixation screw extending at the other side. The last-named type of connections exhibits the same defect as previously mentioned for the pin-hole connection: by the local unnatural loading of the bone resorption occurs, by which a screw or bolt may loosen after some time.

Further perforations of a bone wall give rise to the great disadvantage of inducing a weakening of the naturally heavily loaded walls, which is only compensated under favourable circumstances by growth of the remainder of the cross-section.

The usual further development of the screw principle, according to which a plate is placed at the so-called "periostal" side of the bone wall, also appears to be accompanied by the same problems mentioned.

A disadvantage that occurs with all well-known coupling devices, except for the adjustable internal fixation devices, is that when applying them in children more or less regular reoperating would be necessary to bring the dimensions of the coupling devices into correspondence with the dimensions of the bone having changed during the growth.

Another disadvantage of the prior art orthopedic coupling devices is that they all extend outside the bone so much, that they can seriously disturb and even damage the natural connection and functions of the local connective tissue, muscle tissue, vascular system and so on.

The invention has as an object to provide a solution to the problems occurring in the prior art mentioned and to take away the disadvantages related therewith, and in view thereof provides an orthopedic coupling device for the coupling of a technical element, associated with an endoprothesis with a bone, characterized in that at least one of said legs is provided with at least one projection extending beyond the general surface of said leg in substantially parallel relation with the plane defined by the axes of said legs the device being adapted to be pushed over part of a bone or placed between two parts of bone wall.

It is, in this connection, remarked that CH—A—597 838 describes a generally U-shaped clamp made of resilient material, provided with sharp-edged recesses which serve the purpose of anchoring the device into the bone concerned. That prior art device is adapted to be hammered into the bone. It should be noted that due to the presence of at least one projection according to the invention this clamp could not be hammered into a bone. The clamp according to the invention is specifically meant to be put over a part of the bone or to be put in between two bone parts.

A number of such clamps for instance can be put upon the cortical wall of a broken-off or sawn-off bone. Each of the clamps for example is put upon the bone wall so far, that a firm clamping is obtained on the one side, whilst at the other side between the inside of the U-shape and the bone a free space is kept available, through which a fixation element, for instance a string-line or hook bolt or such, placed at the endoprothesis can be caused to extend.

In using the prior art endoprothesis with stems, the problem often arises that in some situations the necessary length of a long bone for the required length of the stem of the prothesis is not available, so that these prothesis cannot be applied as such. In such a case even bone parts and joints that are in excellent state are also replaced by a technical element. According to the invention this drawback of prior art is largely met; now the bone parts and joints concerned with a small length of the pipe wall can also be saved.

When placing a coupling device according to the invention by pushing it on, the projection concerned scratches an undeep track in e.g. the surface of the cortical wall. In the final position the projections are about stationary with respect to the bone. By this the bone matrix under the projection becomes overloaded, which results in local damaging of the bone matrix: a first small pit is formed. The damaged material will be resorbed. By this moving backward of the bone the pit will widen and the contact stress between projection and bone will diminish gradually, until a natural contact stress is reached, resulting in an excellent mechanical anchorage in a relatively short time. The temporary damage of the membranes of the bone and of the bone itself by the scratching in the cortical bone wall and possibly of the cancellous bone involved, restore themselves very soon, thereby further improving the mechanical anchorage.

The projection mentioned preferably has the shape of a cone with a concave, straight or convex surface, depending on the nature of the fixation. This ascertains a very fast contact at the first touching of the projection and the bone, with a very high initial contact pressure; by the mentioned process of damaging, followed by resorption, the forming of a pit and diminishing of the contact pressure, the fixation is obtained with the desired speed.

In a further embodiment the clamp according to the invention can be provided with a screw extending along one of the legs and adapted to be operated from the outside. Such a screw is of special importance in case of clamping a bone in places where exists a relatively small cortical wall thickness and/or where the projection cannot be applied on its own. The advantage of applying such a screw is the occurrence of a line-shaped series of anchorages of several screw thread windings.

Preferably the screw comprises a cylinder-shaped head having an external thread. Further the screw may comprise a self-tapping part, preferably exhibiting a tapping-groove, lying around the core of the screw as a helix, so that it does not interrupt too much the line-shaped series of anchorages.

Especially advantageous is an embodiment exhibiting the feature according to the invention that the clamp is provided with a threaded hole, the screw is provided with a second cylindrical part provided with thread, the orientation of said thread being the same as that of the self-tapping part.

Advantageously use may be made of an alternative embodiment in which the pitch of the thread of the second cylindrical part is smaller than the pitch of the self-tapping part in such a way, that when turning the screw, the clamp is subjected to a displacement relative to the bone edge corresponding with the difference between the pitches namely when turning the screw on towards the bone edge, to obtain an additional tightening action and when turning the screw loose away from the bone edge, to obtain an additional loosening pushing action.

A preferred embodiment shows the characteristic that at the self-tapping part the outer diameter is constant and the core diameter, in the direction of the leg concerned, directed to the screw is provided with a concave cylindrical curvature, that is adapted to said outer diameter in such a way, that the self-tapping part is over its complete length supported against lateral displacement.

With the measures mentioned above it is achieved that always as many as possible thread windings are in contact with the bone in an as large as possible area, while in addition the tightening action of the screw is promoted as much as possible.

In certain situations it may occur that forces are exercised on the clamp according to the invention, in such a way, that it tends to rotate around the projection. In case of situations in which this is undesirable the invention offers a coupling device in which the inside surface of at least one of the legs of the clamp is bevelled in such a way that it has an inwardly directed cutting edge. To achieve this object an embodiment is also possible in which the legs are tapered towards their free ends. Another advantage of this embodiment is that the flexibility of the clamp increases gradually in the direction of the free ends, while also towards the ends the material is fully loaded, so that a surplus of material is avoided and a better fitting is obtained.

For applications in which the free space between the curve of the clamp and the bone part concerned is difficult to reach, it can be advantageous that the closed side of the U-shape is directed substantially perpendicular to the open side.

According to the invention the material of the clamp will by preference be chosen from the group, compatible with living tissue including: TiALVa (alloy of titanium, aluminum and vanadium) and Vitallium (alloy of among others chrome and cobalt).

The outside of the free end of each of both legs can be bevelled off towards the free end of it and towards the inside. Therewith it is prevented that while pushing up the clamp a spreading force occurs when pushing out the bone material. Namely by the bevelled shape an outwardly directed force upon the clamp is avoided, by which otherwise the clamp can be bent out plastically. Also the force exercised for pushing on is decreased.

The clamp can be manufactured from a material of the type having shape-memory. This shape-memory for example, can be reversible, which is e.g. advantageous in case of an expected necessary renewed fixation or reoperation. Also a material with irreversible shape-memory action may be used, which is advantageous e.g. in case of expected large temperature fluctuations (one can think for example, of visiting a sauna).

It is well-known that the biological properties and growth process of bone are at least partly electrically controlled and therefore can be influenced in an electrical manner. In connection with this it is also well-known that placing conductive materials in the bone, as for instance a metal screw, can cause certain undesirable effects. The invention also objects to provide a solution to this problem and for that purpose provides a coupling device that is supplied with a coating of electrically insulating material, for instance of the ceramic type. This coating may be completely or partly porous, preferably with open pores, in view of a desired tissue ingrowth.

A great flexibility in measure and thus a good versatility is ensured if the legs form part of each a corresponding loose part, said both parts being relatively movable and adapted to be blocked against outward movement in a selectable position. The legs can be adapted to relatively shift, rotate, tilt or hinge. The blocking can be achieved by means of a bolt or screw.

Further features and details of the invention will be mentioned and explained with reference to the drawings.

In the drawing:

Fig. 1a shows a first, simple exemplary embodiment of a clamp according to the invention, positioned over a bone wall;

Fig. 1b shows a top view of the situation shown in Fig. 1a;

Fig. 2 shows a second embodiment of the clamp according to the invention;

Fig. 3a shows a third embodiment of the clamp according to the invention;

Fig. 3b a detail of the clamp according to Fig. 3a;

Fig. 4 shows a fourth embodiment of the clamp according to the invention;

Fig. 5 shows a fifth embodiment of the clamp according to the invention;

Fig. 6a shows a sixth embodiment of the clamp according to the invention;

Fig. 7 shows a seventh embodiment of the clamp according to the invention;

Fig. 8 shows an eighth embodiment of the clamp according to the invention;

Fig. 9a shows a ninth embodiment of the clamp according to the invention;

Fig. 9b shows an example of application of a clamp according to Fig. 9a;

Fig. 10a shows a tenth embodiment of the clamp according to the invention;

Fig. 10b shows a bottom view of the clamp shown in Fig. 10a;

Fig. 10c an example of an embodiment of a screw for a clamp according to the invention;

Fig. 11a shows an eleventh embodiment of a clamp according to the invention;

Fig. 11b shows a bottom view of the clamp shown in Fig. 11a;

Fig. 12 shows a twelfth embodiment of the clamp according to the invention;

Fig. 13 shows an example of application of the clamp shown in Fig. 3a;

Fig. 14a shows a thirteenth exemplary embodiment of a clamp according to the invention, which has the form of an embodiment having shape-memory, before closing the clamp;

Fig. 14b shows the same clamp as in Fig. 14a, but after the closing;

Fig. 15a shows a top view of a bone at the cortical wall of which a number of clamps according to the invention are positioned;

Fig. 16a shows a normal elbow joint;

Fig. 16b shows an example of an elbow prothesis with shafts at both sides;

Fig. 16c shows an elbow joint with a diseased or defective part of bone;

Fig. 16d shows the elbow joint shown in Fig. 16c by which the diseased part of the bone is replaced by an endoprothesis provided with a number of clamps according to the invention.

Fig. 17 shows a perspective view of an endoprothesis that is fixated with a number of clamps according to the invention, to the cortical wall of a bone;

Fig. 18 shows a perspective view in accordance with Fig. 17, in which the joint prothesis is provided with parts extending between the clamps according to the invention.

Fig. 19a a cross-section through a clamp according to a fourteenth example of the invention fixed on the rim of a bone; and

Fig. 19b a front view of the clamp according to Fig. 19a.

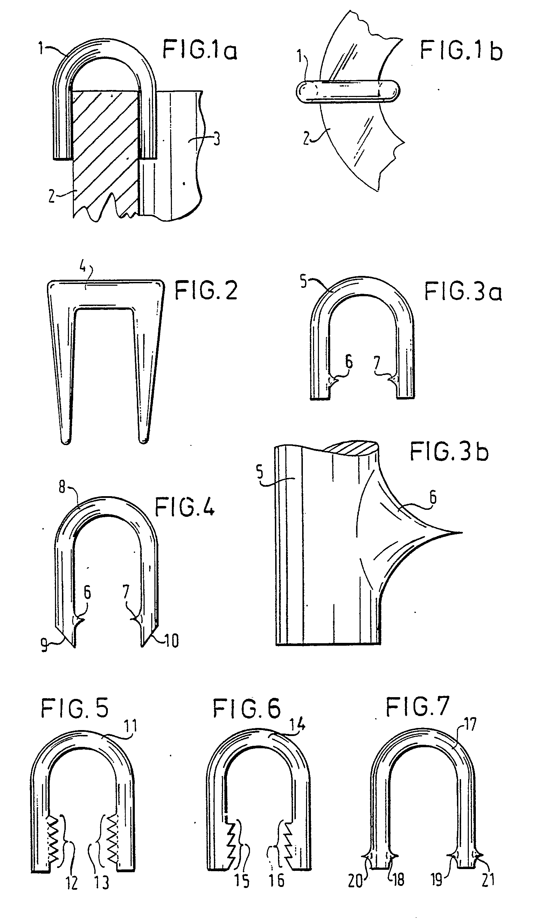

Fig. 1a shows a clamp 1 according to the invention, that is pushed upon a cortical wall 2 of a bone; the cancellous bone is indicated with the reference numeral 3. The clamp 1 shows a U-shape and is constructed as a bent wire with an approximately circular cross-section. A clearer view is given in Fig. 1b showing a top view of the situation shown in Fig. 1.

Fig. 2 shows a second embodiment of the clamp according to the invention. This clamp 4 according to Fig. 2 also shows a general U-shape. Its construction is more rectangular than that of the clamps according to Fig. 1, while besides the legs have a tapered shape towards their free ends. The advantage of this shape is that after placing the clamp forces perpendicular to the main plane of the clamp can also be well carried. Besides it is also advantageous that the flexibilities of the legs increase with increasing distance from the bend of the clamp.

Fig. 3a shows a very important third embodiment of the clamp according to the invention. This clamp 5 is provided with two pointed projections 6 and 7 at its inside near to the free ends of the legs. These projections serve as contact elements between the clamp and the bone part involved. Fig. 3b shows the end of the left leg of the clamp according to Fig. 3a on a larger scale. It may be clearly seen from Fig. 3b that the projection 6 (and also the projection 7) is shaped as a cone having a concave surface. Other shapes are also sometimes desirable, namely for instance straight and convex ones.

Fig. 4 shows a fourth embodiment of the clamp according to the invention. This clamp 8 is, like clamp 5 of the Fig. 3a, provided with projections 6, 7. Unlike clamp 5, clamp 8 shows a shape in which the outside of the free ends of each of both legs is bevelled off towards the free ends and the insides. These bevels are marked in Fig. 4 with reference numbers 9, 10. When pushing the clamp according to Fig. 4 upon a part of bone, the clamp meets an inwardly directed force upon both legs, caused by the presence of the bevels 9 and 10 when penetrating tissue, which prevents spreading that could adversely affect the clamping action.

A fifth exemplary embodiment of the clamp according to the invention is shown in Fig. 5. In this fifth example each of the legs of clamp 11 is provided not only with one projection, but both legs are provided with a number of projections, marked as a group in Fig. 5 with 12, 13 respectively that all extend substantially in the main plane of the U-shape. The projections according to this fifth example have a substantially pointed shape.

Fig. 6 shows a sixth example of the clamp according to the invention. This clamp 14 according to Fig. 6 corresponds for a major part with the clamp 11 according to Fig. 5, in the sense that a number of projections extend in the main plane of the U-shape. The groups of projections 15, 16 of clamp 14 show, unlike those in Fig. 5, a somewhat barb-like shape. Such a shape can appear to be advantageous in those cases in which a very large pulling force must be distributed among more than one tilted projection.

Fig. 7 shows a clamp 17 according to the invention, which is provided on its inside with two projections 18, 19 corresponding with the projections 6, 7 on clamp 5 according to Fig. 3a, and is also provided on its outside with two projections, marked 20, 21 respectively. With this a two-sided anchorage is obtained, after all points have grown fixed, by which in certain situations, such as internal anchorage, there is no danger of the occurrence of spreading or closing forces when pulling the clamp.

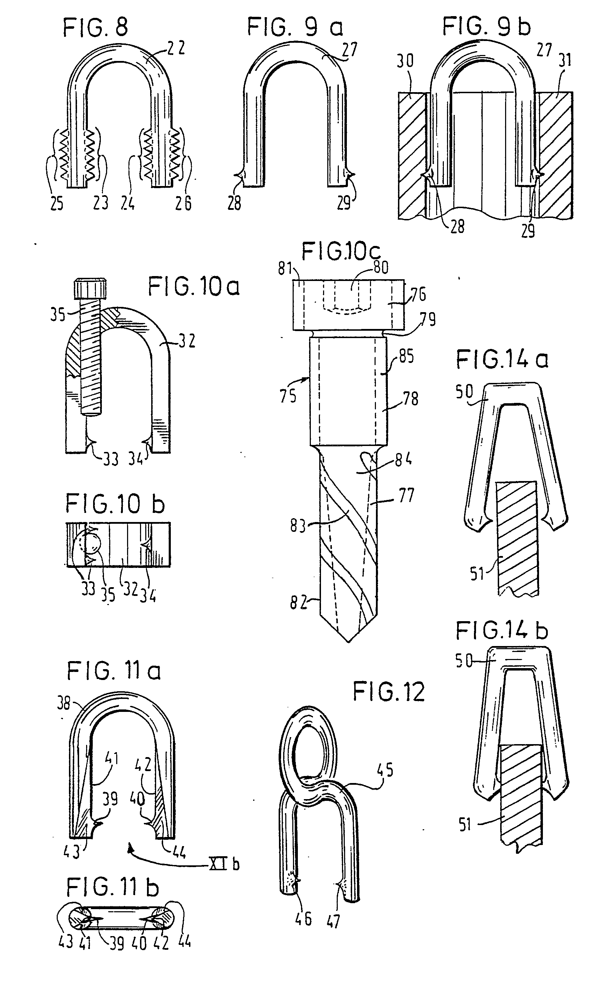

A further development of this principle according to Fig. 7 is displayed in Fig. 8. The clamp 22 according to this figure is supplied with two groups of projections 23, 24 at its inside and with two groups of projections 23, 24 at its outside. Likewise the embodiment according to Fig. 5 can be seen as a further development of the embodiment according to Fig. 3a, the embodiment according to Fig. 8 may be considered as a further development of the example displayed in Fig. 7.

Fig. 9a shows a ninth example of the clamp according to the invention. This clamp 27 is at each of the legs nearby the ends thereof provided with outwardly directed projections 28, 29. This example is especially meant to be placed in between two parts of bone wall, as described schematically in Fig. 9b. In the situation shown in Fig. 9b the clamp 27 is fixed between two parts of cortical wall 30, 31.

Fig. 10a shows a tenth example of the clamp according to the invention. This clamp 32 is provided with one or more inwardly extending projections 33, 34 at each leg and is further provided with a screw 35 extending through the curve of the clamp and along the side of one of the legs to be handled from the outside. By turning on this screw 35 after and/or during the placing of the clamp 32 at and/or towards the desired position an even stronger clamping action and line of fixations is achieved.

Fig. 10b shows a diagrammatic bottom view of the example of Fig. 10a.

Fig. 10c shows an important example of a screw 75 according to the invention. This screw shows four parts in axial direction: a cylindrical head 76, a self-tapping part 77, a cylindrical part 78 and a relatively thin part 79.

The head 76 shows an operating socket 80 and an external screw thread 81, that serves as to further connection or fixation of parts.

The self-tapping part 77 has a constant outer diameter and an inner diameter decreasing towards the end assuring an excellent self-tapping action on the one side and by which on the other side a support is obtained against lateral displacement, because the part 77 is supported along its complete active length by the correspondingly shaped surface (not drawn) of the bone concerned. In the screw coil 82 a tapping slit 83 is made. By the shape described, the part 77 is adapted to tap itself its way through the bone with increasing pressuring by the core 84.

The cylindrical part 78 is provided with an external screw thread 85 for cooperation with a corresponding threaded hole in the clamp.

Fig. 11a shows a clamp 38 according to the invention, having at each of its legs nearby the ends an inwardly directed projection 39, 40. The inside surface of each of the legs of the clamp 38 is bevelled off in such a way that it shows inwardly directed cutting edge, 41, 42 respectively. The bevels, visible from the front are marked with the reference numbers 43, 44. The originating of the cutting edges 41, 42 can be seen more clearly in Fig. 11b which is a bottom view of the clamp 38 according to Fig. 11a.

A twelfth example of the clamp according to the invention is shown in Fig. 12. This clamp 45 which is also provided with two inwardly directed projections 46, 47 respectively is constructed in such a way that the closed side of the U-shape is substantially perpendicular to the open side. Because of this the "eye" can be reached more easily in certain situations, for instance the placing of a bolt.

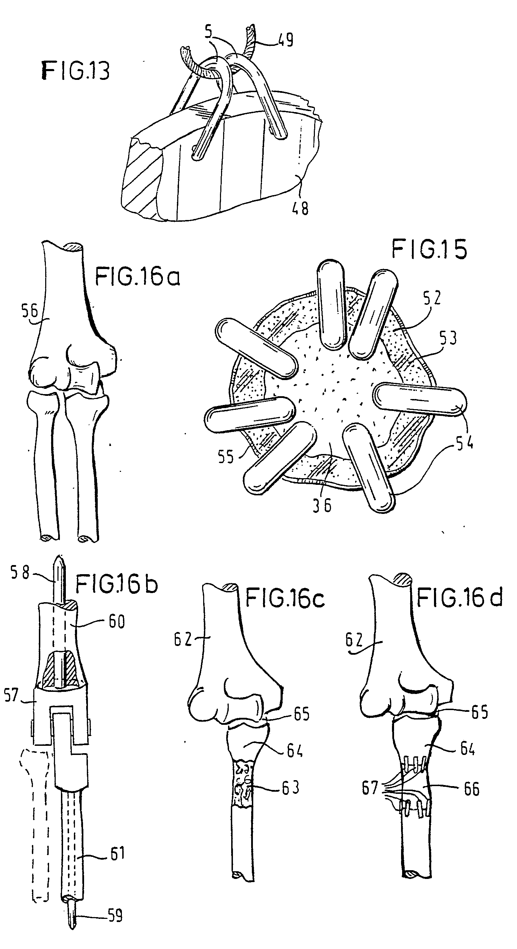

Fig. 13 shows an example of an application, in which two clamps 5 have been placed upon a cortical bone wall 48, while through the eyes formed by the curve of the clamp 5 and the upper edge of the cortex 48 an element is brought having the form of a string-line. In the situation shown, the clamps 5 together are capable of carrying not only longitudinal but also transversal forces, if well-connected to each other.

In this connection it should be noted that some of the examples described are individually capable of carrying transversal forces. These are in particular the clamps 11 according to Fig. 5, 14 according to Fig. 6, 22 according to Fig. 8, 32 according to Fig. 10 and 38 according to Fig. 11.

Fig. 14 shows a clamp according to the invention, being a thirteenth example. This clamp 50 is of the type made of material having shape-memory, in the situation according to Fig. 14a in a spreaded position, as a result of for instance an imposed low temperature, for example about 10°C. This temperature is for instance imposed on it by a pair of tongs, not drawn, that seizes the clamp 50 in good thermal contact. After placing clamp 50 over the cortical bone wall 51, the pair of tongs are loosened from the clamp, which will make it take over the environmental temperature, that will be at about 37°C. By this in a range of temperatures a change occurs in the crystal-structure of the material of the clamp 50, by which it turns from the spreaded form, shown in Fig. 14a, into the elastically clamping form in Fig. 14b. It will be appreciated that with such a clamp not any scratching damage occurs on the bone part concerned, when placing the clamp and that it also can be loosened easily without damage.

Fig. 15 shows a diagrammatic top view of a bone 52, having at its cortical wall 53 placed a number of clamps 54 according to the invention. At the inside of the cortex 53 there is a spongiosa 36 or the cancellous bone, covered with an internal bone membrane or endost and/or a space filled with marrow; at the outside of the cortex there is the external bone membrane or periost 55.

Fig. 16a shows a normal healthy elbow joint marked as a whole with 56. Fig. 16b shows an example of a prior prothesis 57 with at each of both sides a shaft 58, 59. These shafts 58, 59 are pushed into holes, made for that purpose in longitudinal direction in the corresponding bones 60, 61. On the basis of this Fig. 16b it will be clear that in order to obtain an internal grip between the prothesis 57 and the bones 60, 61, by means of the shafts 58, 59, a sufficient length of bone has to be available.

In connection with this, Fig. 16c shows an elbow 62 with a diseased part of bone 63, that is situated rather close to the hinge point of the elbow joint. A comparison with the length of the shafts 58, 59 according to Fig. 16b demonstrates clearly, that the available length of the part of bone 64 between the diseased part of bone 63 and the hinge plane 65 of the joint is so short, that a shaft would receive insufficient support from the bone over that length. Therefore up until now it was usual in such a situation to replace the complete elbow by a prothesis. Because of this a joint that was still good was sacrificed. Another example is the replacement of the knee joint at a too short distal rest of the femur at a femur-endoprothesis.

Fig. 16d shows how the medical problems according to Fig. 16c can be remedied according to the invention. The physician can confine himself to the removal of the diseased part of bone 63 and replace it by an endoprothesis 66 that is coupled by means of a number of clamps 67 according to the invention with natural elasticity and rigidness to the cortical walls of the rest of the bone concerned. In this way the removal of a healthy elbow joint can be prevented.

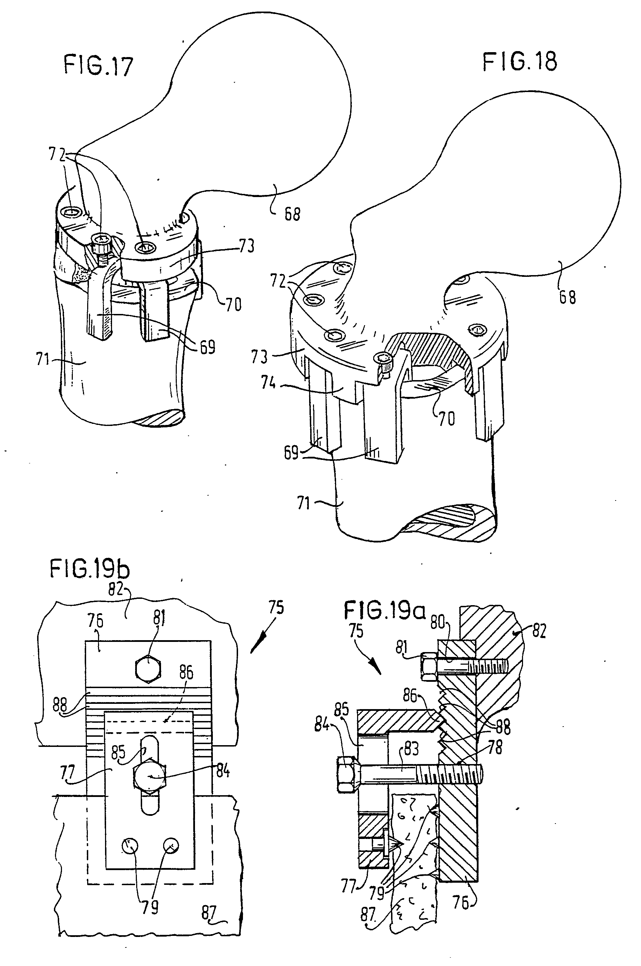

Fig 17 shows a perspective view of an endoprothesis 68 that is fixed with a number of clamps according to the invention to the cortical wall 70 of a bone 71. In this example each of the clamps 69 is provided with a hole or threaded hole, reaching through the curve of the U-shape, through which can be pushed a screw the head of which cooperates with a flange 73, that forms part of the prothesis 68. The situation presented in Fig. 17 shows that there is a free space between the upper side of the cortex and the lower surface of the flange 73 of the prothesis, that can be filled with a preferably elastical filling agent.

Fig. 18 shows a somewhat modified example of the prothesis 68, in which the flange is designed in such a way, that at some places between the clamps there are parts, extending downwards to enclose the filling agent (not to enclose the bone).

Figs. 19a and b show a clamp 75, comprising a first part 76 and a second part 77. The part 76 consists substantially of an elongated plate having a screw hole 78, points 79, and a hole 80 for passing a bolt 81, by which the part 76 is fixed to an endoprothesis 82. The part 77 consists substantially of an L-shaped bent elongated plate having a slot hole 85 for passing a bolt 83 cooperating with said screw hole 78, and points 79. The lower part of the head 84 of said bolt 83 is more or less tapered or convex.

Around the sharp edge 86 the part 77 hinges against a corresponding toothed part 88 of said part 76. The tightened screw 83 blocks the outward relative movement of the parts 76 and 77 in the situation in which clamping takes place on the edge 87 of a bone.

The invention is not restricted to the exemplary embodiments discussed and illustrated with reference to the drawing.

It will be apparent that many modifications and variations may be effected without departing from the scope of the invention. For example the bevels 9, 10 are not restricted to be used at a clamp as according to Fig. 4 only, but for instance can also be applied at a clamp 11 according to Fig. 5. The clamp can have preferably a round cross-section for certain applications, whilst for other applications a more square shape, or a shape with one or more sharp edges may be more convenient. Another embodiment of the clamp according to the invention that has not been discussed extensively, but is obvious after reading of the foregoing description is that by which the clamp has a substantially rectangular construction, while more close to the closed end of the U there are stopping devices to secure always a certain free space between the edge of the part of bone concerned and the clamp; as such a free space is necessary for the fixation of the prothesis concerned. By this it is remarked that such stopping devices are already present in all the designs drawn, for instance in the form of the curve in the U-shape, or in the form of the narrowing shape of the legs towards the closed side of the U.

Also possible is a number of combinations of the principles illustrated before. One can imagine e.g. an embodiment in which more than one projection extends out from the inside of each of the legs, while the outside is supplied with only one projection, that has not necessarily to be positioned exactly opposite one of the projections at the other side of the leg.

In general it is finally remarked that the application of the clamp according to the invention is not restricted to placing upon the cortical wall, but also extends to applying only and/or also in cancellous tissue. Also it will be obvious in connection with this that the dimensions of the clamp have to chosen in dependence with the load carrying capacity of the bone tissue concerned.

## Claims

1. An orthopedic coupling device comprising a generally U-shaped clamp (22) of resilient material having two legs extending in spaced and parallel relation, characterized in that at least one of said legs is provided with at least one projection (23, 24, 25, 26) extending beyond the general surface of said leg in substantially parallel relation with the plane defined by the axes of said legs, the device being adapted to be pushed over part of a bone or placed between two parts of bone wall.

2. The coupling device as claimed in claim 1, characterized in that the projection (6, 7) has the shape of a cone with a concave, convex or straight surface.

3. The coupling device according to claims 1 or 2, characterized in that on each of the legs of the clamp (5) an inwardly directed projection (6, 7) is positioned near the end thereof.

4. The coupling device as claimed in Claims 1, 2, or 3, characterized in that on each of the legs of the clamp (27) an outwardly directed projection (28, 29) is positioned near the end thereof.

5. The coupling device as claimed in any one of the preceding claims, characterized in that the clamp (32) is provided with a screw (35, 75) extending along one of the legs and adapted to be operated from the outside.

6. The coupling device according to claim 5, characterized in that the screw (75) comprises a cylinder-shaped head (76) having an external thread (81).

7. The coupling device according to claim 5 or 6, characterized in that the screw (75) comprises a self-tapping part (77).

8. The coupling device according to claims 5, 6 or 7, characterized in that
the clamp (32) is provided with a threaded hole,
and the screw (75) is provided with a second cylindrical part (78) provided with thread (85), the orientation of said thread (85) being the same as that of the self-tapping part (77).

9. The coupling device according to claim 8, characterized in that the pitch of the thread (85) of the second cylindrical part (78) is smaller than the pitch of the self-tapping part (77) in such a way, that when turning the screw (75), the clamp (32) is subjected to a displacement relative to the bone edge corresponding with the difference between the pitches namely when turning the screw on towards the bone edge, to obtain an additional tightening action and when turning the screw (75) loose away from the bone edge, to obtain an additional loosening pushing action.

10. The coupling device according to claims 6, 7, 8 or 9, characterized in that the outer diameter of the self-tapping part (77) is constant and the core diameter, in the direction of the leg concerned, directed to the screw (75) is provided with a concave cylindrical curvature, that is adapted to said outer diameter in such a way, that the self-tapping part (77) is over its complete length supported against lateral displacement.

11. The coupling device according to any one of the preceding claims, characterized in that the inside surface of at least one of the legs is bevelled off in such a way, that it exhibits an inwardly directed cutting edge (41, 42).

12. The coupling device according to any one of the preceding claims, characterized in that the outside of the free end (9, 10) of each of the legs is bevelled off towards the free end of it and towards the inside.

13. The coupling device according to any one of the preceding claims, characterized in that the legs have a shape tapering towards their free ends.

14. The coupling device according to any one of the preceding claims, characterized in that the closed side of the U-shape is substantially perpendicular to the open side.

15. The coupling device according to any one of the preceding claims, characterized in that the material of the clamp belongs to the group that is compatible with living tissue, whereto belong TiALVa (alloy of titanium, aluminium and vanadium) and Vitallium (alloy of among others chromium and cobalt).

16. The coupling device according to any one of the preceding claims, characterized in that the material of the clamp is of the type having shape-memory.

17. The coupling device according to claim 16, characterized in that the shape-memory is reversible.

18. The coupling device according to claim 16, characterized in that the shape-memory is irreversible.

19. The coupling device according to any one of the preceding claims, characterized by a coating of an electrically insulating material, for example of the ceramic type.

20. A coupling device according to any one of the preceding claims, characterized in that the legs are each formed of a loose part (76, 77), said both parts (76, 77) being relatively movable and adapted to be blocked against outward movement in a selectable position.

21. Technical element (66), e.g. an endoprothesis, characterized by having at least one coupling device (67) according to any one of the preceding claims.

## Patentansprüche

1. Orthopädische Kupplungsvorrichtung mit einer allgemein U-förmigen Klammer (22) aus elastischem Material, die zwei Arme aufweist, die sich im Abstand von einander und parallel zu einander erstrecken dadurch gekennzeichnet, daß mindestens ein Arm mit mindestens einem Vorsprung (23, 24, 25, 26) versehen ist, der über die allgemeine, im wesentlichen parallel zur durch die Achse der Arme bestimmten Ebene verlaufenden Oberfläche hervorragt und die Vorrichtung über einen Teil eines Knochens oder zwischen zwei Teile einer Knochenwand eingeschoben werden kann.

2. Kupplung nach Anspruch 1 dadurch gekennzeichnet, daß der Vorsprung (6, 7) die Form eines Kegels mit konkaver, konvexer oder gerader Oberfläche hat.

3. Kupplung nach Anspruch 1 oder 2 dadurch gekennzeichnet, daß an jedem Arm der Klammer (5) ein nach innen gerichteter Vorsprung (, 7) in der Nähe des freien Endes des Armes vorgesehen ist.

4. Kupplung nach Anspruch 1, 2 oder 3 dadurch gekennzeichnet, daß an jedem Arm der Klammer (27) ein nach außen gerichteter Vorsprung (28, 29) nahe dem freien Ende des Armes angeordnet ist.

5. Kupplung nach einem der vorhergehenden Ansprüche dadurch gekennzeichnet, daß die Klammer (32) mit einer Schraube (35, 75) versehen ist, die sich entlang eines der Arme erstreckt und von außen bedienbar ist.

6. Kupplung nach Anspruch 5 dadurch gekennzeichnet, daß die Schraube (75) einen zylindrischen Kopf (76) mit äußerem Gewinde (81) aufweist.

7. Kupplung nach Anspruch 5 oder 6 dadurch gekennzeichnet, daß die Schraube (75) einen selbsttätig eindringenden Teil (77) aufweist.

8. Kupplung nach Anspruch 5, 6 oder 7 dadurch gekennzeichnet, daß die Kupplung (32) mit einer Gewindebohrung versehen ist und die Schraube (75) mit einem zweiten zylindrischen Teil (78) mit Gewinde (85) versehen ist, wobei die Ausrichtung des Gewindes (85) die gleiche ist wie die des selbsttätig eindringenden Teils (77).

9. Kupplung nach Anspruch 8 dadurch gekennzeichnet, daß die Steigung des Gewindes (85) des zweiten zylindrischen Teils (78) geringer ist als die Steigung des selbst eindringenden Teils (77), sodaß bei Drehung der Schraube (75) die Kupplung (32) relativ zur Knochenkante entsprechend dem Steigungsunterschied zwischen den beiden Gewinden versetzt wird, wenn die Schraube in Richtung auf die Knochenkante angezogen wird, um eine zusätzliche Anspannung und bei Drehung der Schraube (75) hinweg von der Knochenkante bewegt wird, um eine zusätzliche Freigabebewegung zu erreichen.

10. Kupplung nach Anspruch 6, 7, 8 oder 9 dadurch gekennzeichnet, daß der äußere Durchmesser des selbsteindringenden Teils (77) konstant ist und der Kerndurchmesser in Richtung des betroffenen Armes, der zur Schraube (75) gerichtet ist, mit einer zylindrischen Konkavform versehen ist, die in der Weise an den äußeren Durchmesser angepasst ist, daß der selbsteindringende Teil (77) über seine ganze Länge gegen seitliche Versetzungen abgestützt wird.

11. Kupplung nach einem der vorhergehenden Ansprüche dadurch gekennzeichnet, daß die innere Oberfläche zumindest eines der Arme so abgeschrägt ist, daß sie eine nach innen gerichtete Schneidkante (41, 42) zeigt.

12. Kupplung nach einem der vorhergehenden Ansprüche dadurch gekennzeichnet, daß die Außenseite des freien Endes (9, 10) jedes Armes zum freien Ende und nach der Innenseite hin abgeschrägt ist.

13. Kupplung nach einem der vorhergehenden Ansprüche dadurch gekennzeichnet, daß die Arme zu ihren freien Enden hin kegelförmig zulaufen.

14. Kupplung nach einem der vorhergehenden Ansprüche dadurch gekennzeichnet, daß die geschlossene Seite der U-Form im wesentlichen rechtwinklig zur offenen Seite verläuft.

15. Kupplung nach einem der vorhergehenden Ansprüche dadurch gekennzeichnet, daß das Material der Klammer zu einer Gruppe gehört, die mit lebendigem Gewebe verträglich ist, wozu TIALVA (Legierungen aus Titan, Aluminium und Vanadium) und Vitallium (Legierung unter anderem mit Chrom und Kobalt) gehören.

16. Kupplung nach einem der vorhergehenden Ansprüche dadurch gekennzeichnet, daß das Material der Klammer ein Formgedächtnis aufweist.

17. Kupplung nach Anspruch 16 dadurch gekennzeichnet, daß das Formgedächtnis umkehrbar ist.

18. Kupplung nach Anspruch 16 dadurch gekennzeichnet, daß das Formgedächtnis irreversibel ist.

19. Kupplung nach einem der vorhergehenden Ansprüche dadurch gekennzeichnet, daß ein Überzug aus elektrisch isolierendem Material z.B. Keramik vorgesehen ist.

20. Kupplung nach einem der vorhergehenden Ansprüche dadurch gekennzeichnet, daß jeder Arm von einem losen Teil (76, 77) gebildet wird, die beide verschiebbar und gegen eine Bewegung nach außen in auswählbarer Stellung blockierbar sind.

21. Technisches Element (66) z.B. eine Endoprothese dadurch gekennzeichnet, daß sie mindestens eine Kupplungsvorrichtung (67) nach einem der vorhergehenden Ansprüche aufweist.

**Revendications**

1. Dispositif d'accouplement orthopédique comprenant une attache (22) de forme générale en U en matériau élastique ayant deux jambes espacées et s'étendant parallèlement, caractérisé en ce qu'au moins une desdites jambes est munie d'au moins une partie en saillie (23, 24, 25, 26) s'étendant au delà de la surface générale de ladite jambe à peu près parallèlement au plan défini par les axes desdites jambes, le dispositif étant adapté pour être disposé au dessus d'une partie d'un os ou entre des parties d'une paroi osseuse.

2. Dispositif d'accouplement selon la revendication 1, caractérisé en ce que la partie faisant saillie (6, 7) se présente sous la forme d'un cône avec une surface concave, convex ou droite.

3. Dispositif d'accouplement selon l'une quelconque des revendications 1 ou 2, caractérisé en ce qu'une partie faisant saillie (6, 7) dirigée vers l'intérieur est positionnée sur chaque jambe de l'attache (5) près de l'extrémité de celle-ci.

4. Dispositif d'accouplement selon l'une quelconque des revendications 1, 2 ou 3, caractérisé en ce qu'une partie faisant saillie (28, 29) dirigée vers l'extérieur est positionnée sur chacune des jambes de l'attache (27) près de l'extrémité de celle-ci.

5. Dispositif d'accouplement selon l'une quelconque des revendications précédentes, caractérisé en ce que l'attache (32) est munie d'une vis (35, 75) s'étendant le long d'une des jambes et étant adaptée pour être manoeuvrée de l'extérieur.

6. Dispositif d'accouplement selon la revendication 5, caractérisé en ce que la vis (75) comprend une tête de forme cylindrique (76) ayant un filet extérieur (81).

7. Dispositif d'accouplement selon la revendication 5 ou 6, caractérisé en ce que la vis (75) comprend une partie auto-taraudeuse (77).

8. Dispositif d'accouplement selon la revendication 5, 6 ou 7, caractérisé en ce que l'attache (32) est munie d'un trou fileté et en ce que la vis (75) est munie d'une seconde partie cylindrique (78) comportant un filet (85), dont l'orientation est la même que celle de la partie auto-taraudeuse (77).

9. Dispositif d'accouplement selon la revendication 8, caractérisé en ce que la pas du filet (85) de la seconde partie cylindrique (78) est plus petit que le pas de la partie auto-taraudeuse (77) de manière que lorsque l'on tourne la vis (75), l'attache (32) est soumise à un déplacement par rapport au bord de l'os qui correspond à la différence entre les pas en particulier, lorsque l'on tourne la vis vers le bord de l'os pour obtenir une action de serrage supplémentaire et lorsque l'on tourne la vis (75) pour l'éloigner du bord de l'os pour obtenir une action de poussée en relachement supplémentaire.

10. Dispositif d'accouplement selon les revendications 6, 7, 8 ou 9, caractérisé en ce que la diamètre extérieur de la partie auto-taraudeuse (77) est constant et en ce que ledit diamètre du noyau, dans la direction de la jambe concernée, orienté vers la vis (75), est prévue avec une courbure cylindrique concave, qui est adaptée audit diamètre extérieur de manière que la partie auto-taraudeuse (77) est supportée contre tout déplacement latéral sur sa longueur totale.

11. Dispositif d'accouplement selon l'une quelconque des revendications précédentes, caractérisé en ce que la surface interne d'au moins une des jambes est biseautée de manière qu'elle présente un bord coupant dirigé vers l'intérieur (41, 42).

12. Dispositif d'accouplement selon l'une quelconque des revendications précédentes, caractérisé en ce que l'extérieur de l'extrémité libre (9, 10) de chacune des jambes est biseauté vers l'extrémité libre de celle-ci et vers l'intérieur.

13. Dispositif d'accouplement selon l'une quelconque des revendications précédentes, caractérisé en ce que les jambes présentent une forme rétrécie vers leurs extrémités libres.

14. Dispositif d'accouplement selon l'une quelconque des revendications précédentes, caractérisé en ce que le côté fermé de la forme en U est à peu près perpendiculaire au côté ouvert.

15. Dispositif d'accouplement selon l'une quelconque des revendications précédentes, caractérisé en ce que le matériau constituant l'attache appartient au groupe qui est compatible avec les tissus vivants, c'est-à-dire TiAlVa (alliage de titane-aluminium et vanadium) et vitallium (alliages parmi d'autres de chrome et de cobalt).

16. Dispositif d'accouplement selon l'une quelconque des revendications précédentes, caractérisé en ce que le matériau constituant l'attache est du type ayant une mémoire de forme.

17. Dispositif d'accouplement selon la revendication 16, caractérisé en ce que la mémoire de forme est réversible.

18. Dispositif d'accouplement selon la revendication 16, caractérisé en ce que la mémoire de forme est irréversible.

19. Dispositif d'accouplement selon l'une quelconque des revendications précédentes, caractérisé en ce qu'il comporte un revêtement de matériau isolant électriquement, par exemple du type céramique.

20. Dispositif d'accouplement selon l'une quelconque des revendications précédentes, caractérisé en ce que les jambes sont formées chacune d'une partie séparable (76, 77) cesdites deux parties (76, 77) étant relativement mobiles et adaptées pour être bloquées contre tout mouvement vers l'extérieur dans une position pouvant être sélectionné.

21. Elément technique (66) par exemple une endoprothèse, caractérisé en ce qu'il comporte au moins un dispositif d'accouplement (67) selon l'une quelconque des revendications précédentes.

FIG.1a

FIG.1b

FIG.2

FIG.3a

FIG.4

FIG.3b

FIG.5

FIG.6

FIG.7

1

**0 040 884**

FIG. 8

FIG. 9 a

FIG. 9 b

FIG.10c

FIG. 10 a

FIG.14 a

FIG. 10 b

FIG.14 b

FIG. 11 a

FIG. 12

FIG. 11 b

2

**0 040 884**

FIG.13

FIG.16a

FIG.15

FIG.16b

FIG.16c

FIG.16d

FIG.17  FIG.18

FIG.19b

FIG.19a